(19) Europäisches Patentamt
European Patent Office
Office européen des brevets

(11) Numéro de publication: **0 203 902**
**A2**

(12) # DEMANDE DE BREVET EUROPEEN

(21) Numéro de dépôt: **86870064.2**

(22) Date de dépôt: **05.05.86**

(51) Int. Cl.⁴: **C 07 D 487/04,** A 61 K 31/55
// (C07D487/04, 223:00, 209:00)

(30) Priorité: **10.05.85 LU 85894**

(43) Date de publication de la demande: **03.12.86**
**Bulletin 86/49**

(84) Etats contractants désignés: **AT BE CH DE FR GB IT LI LU NL SE**

(71) Demandeur: **OMNICHEM Société anonyme, 12 Avenue de Broqueville, B-1150 Bruxelles (BE)**

(72) Inventeur: **Hannart, Jean-Alfred Alphonse, 25, Avenue d'El Pirere, B-1302 Dion-Valmont (BE)**
Inventeur: **Dejonghe, Jean-Paul, 94, rue Ch.Jaumotte, B-1350 Wavre (BE)**
Inventeur: **De Campeneer, Danielle, 10, avenue Jean de Luxembourg, B-1330 Rixensart (BE)**
Inventeur: **Maloteaux, Jean-Marie, 633, Chausée de Villers, B-6310 Les Bons Villers (BE)**

(74) Mandataire: **Van Malderen, Michel et al, p.a. Freylinger & Associés 22 avenue J.S. Bach (bte 43), B-1080 Bruxelles (BE)**

(54) **Dérivés de 1,2,3,4,5,6-hexahydro-azépino[4,5-b]indole, leur préparation, composes intermédiaires, et leur application en thérapeutique.**

(57) L'invention est relative à de nouveaux dérivés de 1,2,3,4,5,6-hexahydro-5-hydroxy-alkyl-azépino(4,5-b) indole de formule:

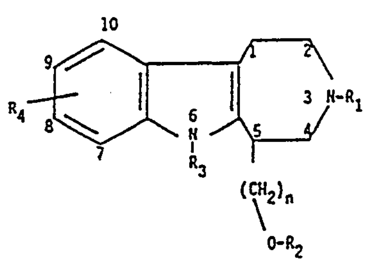

I

dans laquelle
$R_1$ représente un atome d'hydrogène, un radical alkyle, un radical alcényle, un radical benzyle, un radical alkylamino du type 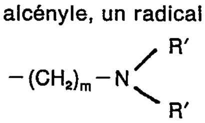
où les groupes R′ sont soit des atomes d'hydrogène, soit des radicaux alkyle ou forment ensemble avec l'atome d'azote auxquels ils sont attachés un noyau hétérocycle du type morpholinyle, pipéridinyle, pyrrolidinyle, pipérazinyle et m est 2 ou 3,
$R_2$ représente un atome d'hydrogène, ou un radical benzoyle ou acyle,
$R_3$ représente un atome d'hydrogène, un radical alkyle ou benzyle,
$R_4$ représente un atome d'hydrogène ou d'halogène, un radical aklyle, un radical alcoxy, un radical trifluorométhyle, et n est 1 ou 2 et les sels d'addition d'acides pharmaceutiquement acceptables et à des procédés pour leur préparation, à des composés intermédiaires et à l'application thérapeutique desdits dérivés.

EP 0 203 902 A2

86870064.2

0203902

# DERIVES DE 1,2,3,4,5,6-HEXAHYDRO-AZEPINO [4,5-b] INDOLE, LEUR PREPARATION, COMPOSES INTERMEDIAIRES, ET LEUR APPLICATION EN THERAPEUTIQUE.

La présente invention concerne des dérivés de 1,2,3,4,5,6-hexahydro-azépino [4,5-b] indole, leur préparation et leur application en thérapeutique. Elle s'étend également aux produits intermédiaires obtenus au cours de leur préparation.

La présente invention concerne les compositions pharmaceutiques ayant pour objet des dérivés de 1,2,3,4,5,6-hexahydro-5-hydroxyalkyl-azépino [4,5-b] indole éventuellement sous forme de sels d'addition à un acide thérapeutiquement acceptable répondant à la formule I

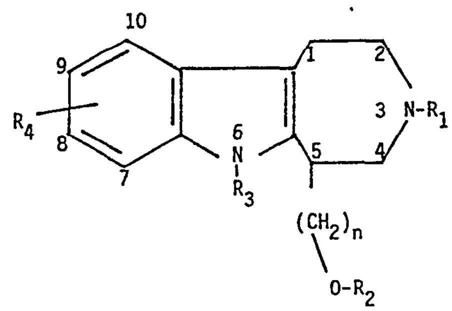

I

dans laquelle n est 1 ou 2.

$R_1$ représente un atome d'hydrogène, un radical alkyle, un radical alcényle, un radical benzyle, un radical alkylamino du type $-(CH_2)_m-N\begin{matrix}R'\\R'\end{matrix}$

où les groupes R' sont soit des atomes d'hydrogène, soit des radicaux alkyles ou forment ensemble avec l'atome d'azote auquel ils sont attachés, un noyau hétérocycle du type morpholinyle, pipéridinyle, pyrrolidinyle, pipérazinyle et m est 2 ou 3.

$R_2$ représente un atome d'hydrogène, un radical benzoyle ou acyle.

$R_3$ représente un atome d'hydrogène, un radical alkyle ou benzyle.

$R_4$ représente un atome d'hydrogène ou d'halogène, un radical alcoxy, un radical trifluorométhyle.

Les termes "alkyle" et "alcoxy" ont de 1 à 4 atomes de carbone et le terme "acyle" de 2 à 4 atomes de carbone.

Parmi les dérivés susmentionnés, on peut tout particulièrement citer :

le 1,2,3,4,5,6-hexahydro-5-hydroxyméthyl-azépino [4,5-b] indole,

le 1,2,3,4,5,6-hexahydro-3-méthyl-5-hydroxyméthyl-azépino [4,5-b] indole,

le 1,2,3,4,5,6-hexahydro-3-éthyl-5-hydroxyméthyl-azépino [4,5-b] indole,

le 1,2,3,4,5,6-hexahydro-3-propyl-5-hydroxyméthyl-azépino [4,5-b] indole,

le 1,2,3,4,5,6-hexahydro-5-hydroxyméthyl-6-méthyl-azépino [4,5-b] indole,

le 1,2,3,4,5,6-hexahydro-3,6-diméthyl-5-hydroxyméthyl-azépino [4,5-b] indole,

le 1,2,3,4,5,6-hexahydro-3-éthyl-5-hydroxyméthyl-6-méthyl-azépino [4,5-b] indole,

le 1,2,3,4,5,6-hexahydro-3-méthyl-5-hydroxyméthyl-6-éthyl-azépino [4,5-b] indole,

le 1,2,3,4,5,6-hexahydro-3-éthyl-5-hydroxyéthyl-azépino [4,5-b] indole.

La matière première de départ pour la préparation des dérivés 1 où n = 1 est le 1,2,3,4,5,6-hexahydro-5-carboalkoxy-azépino [4,5-b] indole de formule IIa

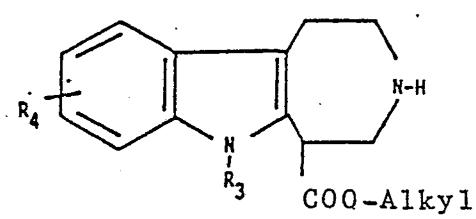

IIa

où $R_3$ et $R_4$ ont la signification mentionnée ci-dessus.

Différentes approches de synthèse pour la préparation du produit IIa ont été décrites par Kuehne dans les brevets suivants : brevet U.S. n°4.154.843 du 15 mai 1979, brevet U.S. n°4.267.330 du 12 mai 1981, brevet U.S. n°4.283.536 du 11 août 1981, brevet U.S. n°4.362.739 du 7 décembre 1982.

La matière première de départ pour la préparation des dérivés I où n = 2 est le 1,2,3,6, tétrahydro-5-carbométhoxy - méthylène-azépino [ 4,5-b] indole 4-one de formule IIb

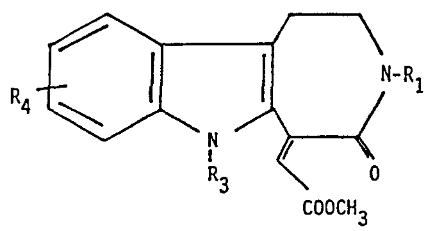

IIb

où $R_1$, $R_3$, $R_4$ ont la signification mentionnée ci-dessus.

Ces produits peuvent être préparés à partir de tétrahydro β carboline substituée.

Les composés I, où n = 1, de la présente invention peuvent être préparés selon deux procédés.

Premier procédé.

Ce procédé comprend d'abord la réduction du groupement carboalkoxy du produit IIa, suivie de la substitution de l'azote en position 3, selon le schéma général suivant :

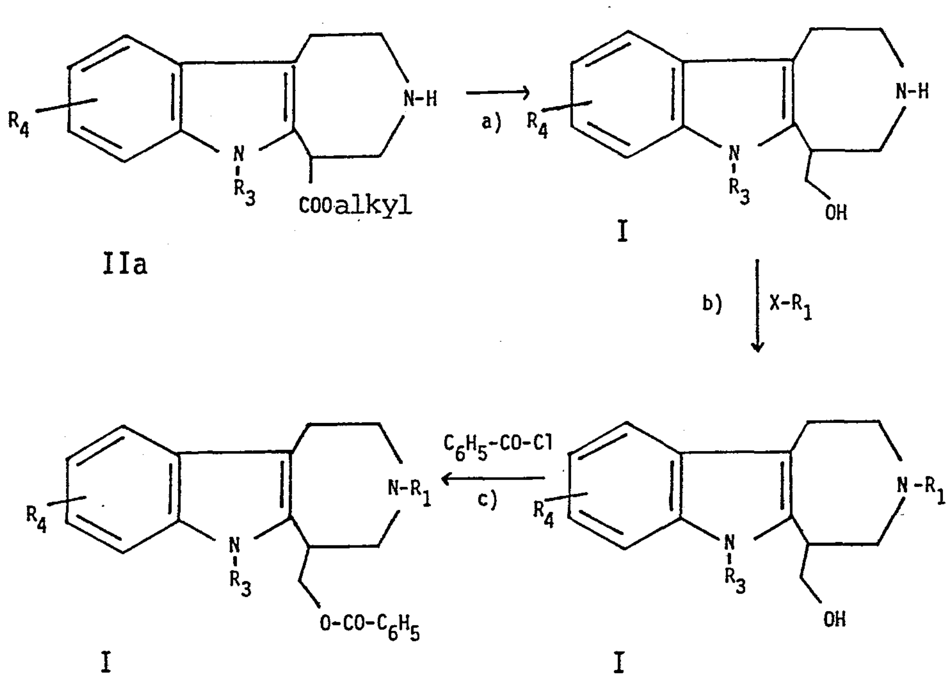


IIa I I I

où $R_1$, $R_3$, $R_4$ ont la signification mentionnée ci-dessus, X représente un atome d'halogène.

L'étape a) de réduction du groupement carboalkoxy du composé IIa peut être effectuée dans un solvant organique inerte, par exemple le tétrahydrofuranne. La température de réaction peut être comprise entre 20°C et la température de reflux du solvant. L'agent de réduction peut être l'hydrure de lithium aluminium.

L'étape b) d'alkylation peut être effectuée d'une manière conventionnelle par condensation d'un halogénure d'alkyle, de benzyle ou d'alkylamino substitué ou non, en présence d'une amine tertiaire telle que la diisopropyl éthylamine ou la triéthylamine dans un hydrocarbure aliphatique chloré tel que le chloroforme ou le dichloroéthane. La température de la réaction peut être comprise entre 20°C et la température de reflux du solvant.

L'étape c) de benzoylation ou d'acylation peut être effectuée d'une manière conventionnelle par condensation du chlorure de benzoyle ou d'un chlorure d'acide en présence de pyridine dans un hydrocarbure aliphatique chloré tel que le chloroforme ou le dichloroéthane à la température ambiante.

Second procédé.

Ce procédé comprend d'abord la substitution de l'azote en position 3 du produit IIa, suivie de la réduction du groupement carbométhoxy selon le schéma suivant :

1) Préparation de dérivés I où R1 est un radical alkyle, un radical alcényle, un radical benzyle.

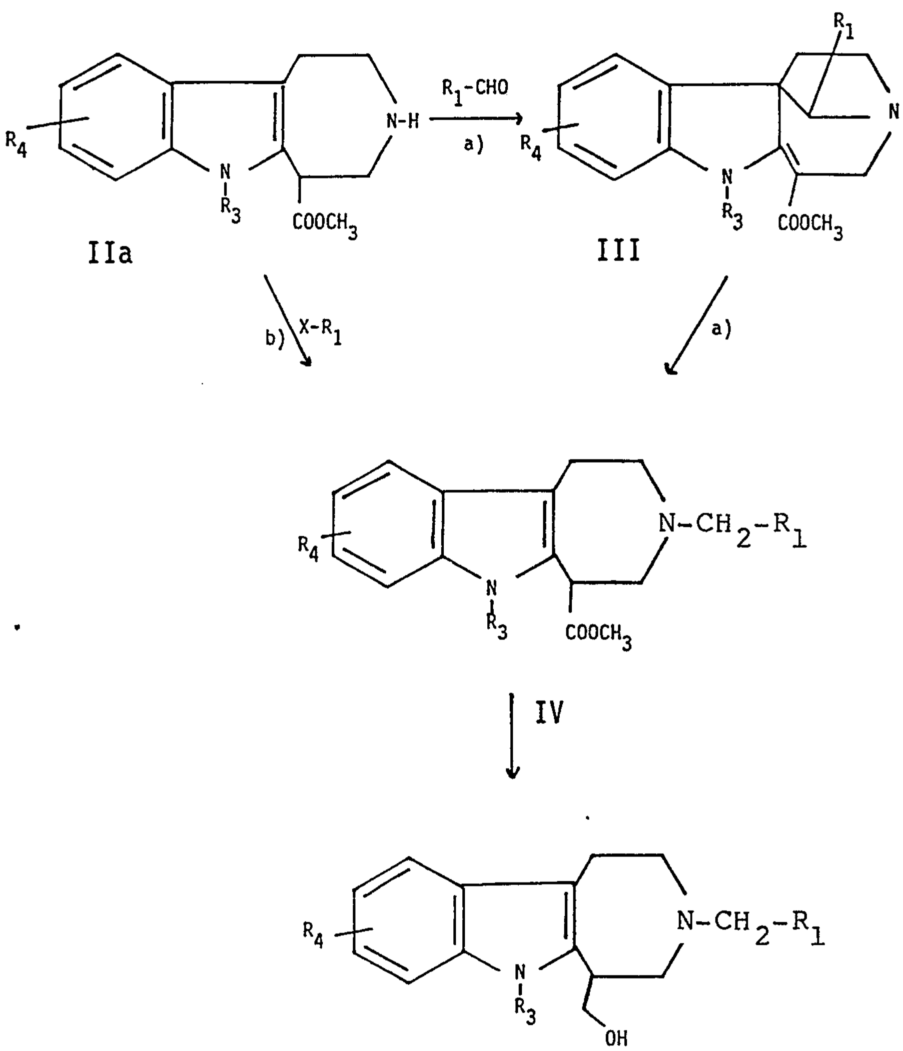


IIa

III

IV

I

où $R_3$, $R_4$ ont la signification mentionnée ci-dessus.

Le procédé a) fait intervenir la condensation d'un aldéhyde aliphatique ou aromatique avec le produit IIa, pour former les méthanoazépinoindoles de formule III, suivant la technique décrite par Kuehne dans le brevet U.S. n°4.362.739 du 7 décembre 1982. La réduction de III en IV peut être effectuée dans un solvant organique tel que le méthanol ou l'éthanol en milieu acide, à la température ordinaire. L'agent de réduction peut être le cyanoborohydrure de sodium.

Le procédé b) fait intervenir la condensation d'un halogénure d'alkyle, d'alcényle, de benzyle, avec le produit IIa, pour former le produit IV. La réaction de condensation peut être réalisée d'une manière conventionnelle en présence d'une amine tertiaire telle que la diisopropyléthylamine ou la triéthylamine, dans un hydrocarbure aliphatique chloré tel que le chloroforme ou le dichloroéthane.
Le procédé b) peut faire intervenir également la condensation d'un chloroformiate d'alkyle ou un chlorure d'acide. Dans ce cas, la réaction est réalisée dans un système binaire composé d'une solution du produit IIa dans l'acétate d'éthyle et d'une solution aqueuse de soude caustique, pour donner le produit IV où $R_1$ est un groupement -COO-Alkyl ou -CO-Alkyl.

La réduction du produit IV en produit I est réalisée suivant la technique décrite dans le premier procédé par l'hydrure de lithium aluminium.

2) Préparation de dérivés I ou R1 est un radical alkylamino.

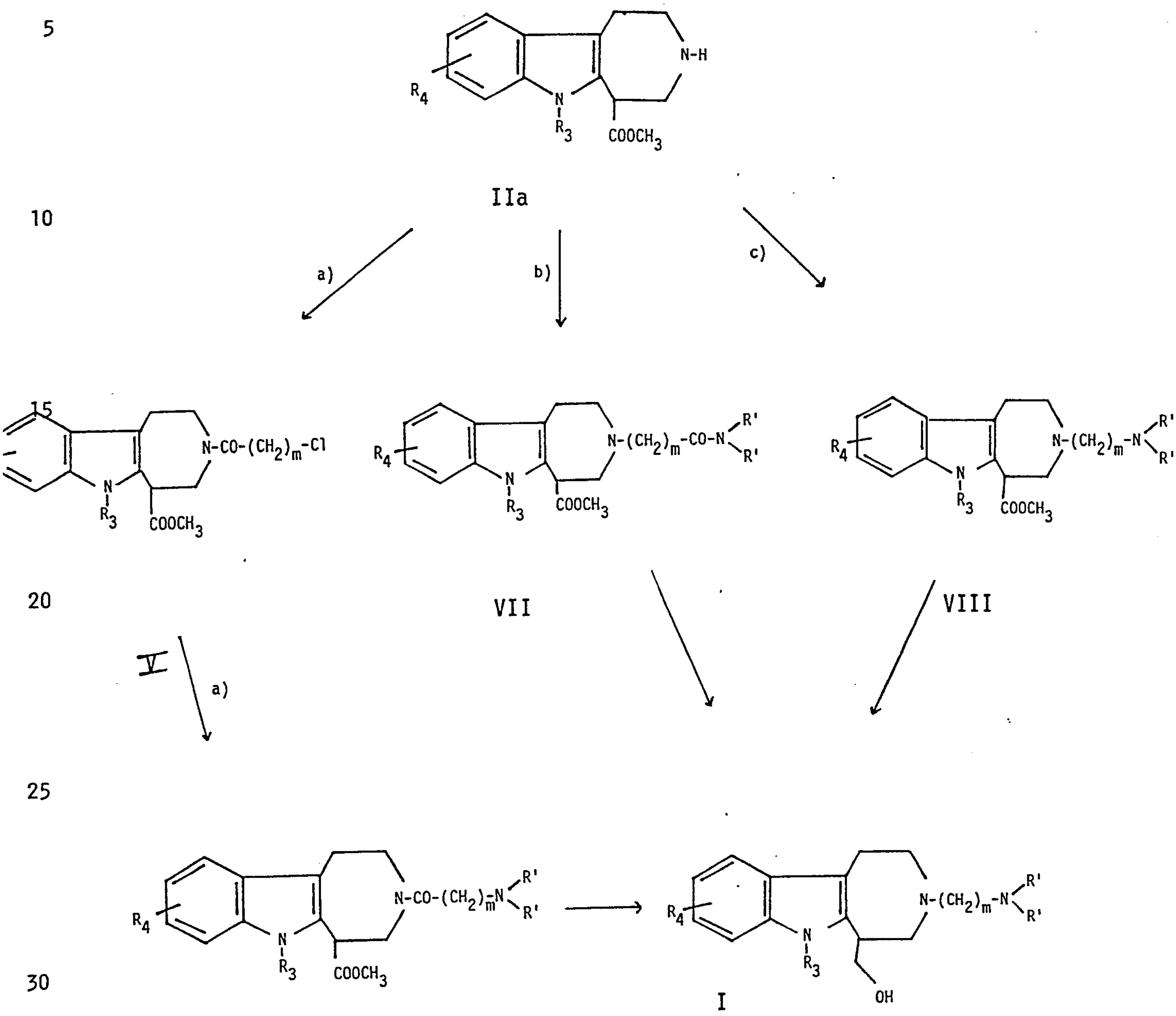


IIa

VII

VIII

VI

I

où $R_3$, $R_4$ ont les significations mentionnées ci-dessus.

Le procédé a) est réalisé par l'addition d'un chlorure d'acide de type $X-(CH_2)_m-COCl$ où X est un atome de brome ou de chlore et m = 1 ou 2, à un système binaire composé d'une solution du produit IIa dans l'acétate d'éthyle et d'une solution aqueuse de soude caustique. Le produit V est ensuite condensé avec des amines primaires ou secondaires dans un solvant organique tel que le tétrahydrofuranne, pour donner le produit VI.

Le procédé b) est réalisé par l'addition de chloroamides de type $Cl-(CH_2)_m-CO-N<{R' \atop R'}$ où m = 1 ou 2, au produit IIa en présence d'une amine tertiaire telle que la diisopropyléthylamine ou la triéthylamine, soit dans un hydrocarbure aliphatique chloré tel que le chloroforme ou le dichloroéthane, soit en présence de carbonate de potassium dans la méthyléthylcétone, pour donner le produit VII.

Le procédé c) est réalisé d'une manière conventionnelle par condensation d'un halogénure d'alkylamine de type $X-(CH_2)_m-N<{R' \atop R'}$ où X est un atome de chlore ou de brome et m = 2 ou 3, au produit IIa en présence d'une amine tertiaire telle que la diisopropyléthylamine ou la triéthylamine, dans un hydrocarbure aliphatique chloré tel que le chloroforme ou le dichloroéthane, pour donner le produit VIII.

La réduction des produits VI, VII et VIII en produit I est réalisée suivant la technique décrite dans le premier procédé, par l'hydrure de lithium aluminium.

Les composés I où n = 2 de la présente invention peuvent être préparés selon le procédé suivant :

Le procédé comprend d'abord la réduction catalytique du groupement carboxyméthylène du produit IIb en produit IX, suivie de la réduction du groupement carbométhoxy en produit I, selon le schéma général suivant :

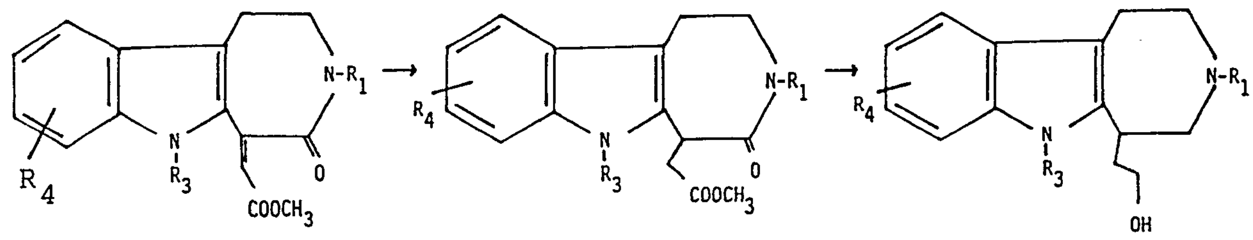

IIb IX I

La réduction de IIb peut être effectuée d'une manière conventionnelle par une hydrogénation catalytique au moyen de charbon palladié. L'hydrogénation catalytique peut être effectuée dans un solvant approprié, par exemple l'acide acétique à des températures comprises entre 20°C et 40°C. La réduction du groupement carbométhoxy et du groupement oxo est réalisée suivant la technique décrite dans le premier procédé par l'hydrure de lithium aluminium.

L'étude pharmacologique des composés de l'invention montre qu'ils sont actifs dans trois épreuves d'hypoxie : hypoxie hypobare, hypoxie histotoxique induite au KCN et hypoxie normobare à l'azote.

Les composés de l'invention possédant une activité antianoxique peuvent être utilisés en thérapeutique pour le traitement des troubles de la vigilance, en particulier pour lutter contre les troubles du comportement imputables à des dommages vasculaires cérébraux et à la sclérose cérébrale en gériatrie.

L'invention comprend par conséquent toutes compositions pharmaceutiques renfermant les composés et/ou leurs sels comme principes actifs, en association avec tous excipients appropriés à leur administration, en particulier par voie orale ou parentérale.

La posologie quotidienne peut aller de 10 à 100 mg.

Les exemples suivants illustrent de manière non limitative les caractéristiques de l'invention.

Exemple 1 : 1,2,3,4,5,6-hexahydro-5-hydroxyméthyl-azépino [4,5-b] indole
(I: $R_1$, $R_2$, $R_3$, $R_4$ = H; n = 1)

A une suspension de 3,9 g (0,102 m) d'hydrure de lithium aluminium dans 250 ml de tétrahydrofuranne, est ajouté goutte à goutte sous argon, une solution de 24,43 g (0,1 m) de 5-carbométhoxy azépino [4,5-b] indole dissous dans 300 ml de tétrahydrofuranne.
Le mélange réactionnel est agité 2 heures à température ambiante. Il est ensuite traité par du sulfate de magnésium saturé en eau. Le mélange est agité une heure et ensuite filtré sur dicalite. Le précipité est rincé par 50 ml de tétrahydrofuranne.
Le filtrat est ensuite évaporé sous vide et le résidu est repris dans 300 ml de chlorure de méthylène, lavé deux fois par une solution saturée en sel et séché sur sulfate de magnésium. Après filtration et évaporation, on obtient 15,6 g de matière organique qu'on purifie par chromatographie sur silice (élution $CH_2Cl_2$/MeOH 2%). Le résidu est cristallisé dans le méthanol et donne 12,9 g de produit.
Rdt : 60%.

Fus : 149°C

U.V. (MeOH) $\lambda_{max}$: 224, 282, 291 nm

S.M. m/e (%) : 216 (89 $M^+$), 198 (16), 186 (28), 174 (92), 168 (17), 156 (100), 144 (56), 130 (33), 115 (14).

I.R. (KBr) : 3300, 2900, 1623, 1462, 1347, 1094, 1022, 831 $cm^{-1}$

R.M.N. ($CDCl_3$)δ: 8,56 (m, 1H), 7,46-6,76 (m, 4H), 3,73 (m, 4H), 3,33-2,43 (m, 7H).

Exemple 2 : 1,2,3,4,5,6-hexahydro-3-méthyl-5-hydroxyméthyl-azépino [4,5-b]indole. (I : $R_1$ = $CH_3$; $R_2$, $R_3$, $R_4$ = H; n = 1)

a) 1,2,3,4,5,6-hexahydro-3,5-dicarbométhoxy-azépino [4,5-b] indole (IV : $R_1$ = $COOCH_3$; $R_3$, $R_4$ = H)

A une solution de 6,1 g (0,025 m) de 5-carbométhoxyazépino[4,5-b]indole dans 100 ml d'acétate d'éthyle, on ajoute 80 ml de soude caustique 2 M et sous bonne agitation 3,543 g (0,0375 m) de chloroformiate de méthyle en solution dans 10 ml d'acétate d'éthyle. Après 10 minutes d'agitation, la réaction est terminée. On décante la phase organique et on extrait deux fois la phase aqueuse par 50 ml d'acétate d'éthyle. Les phases organiques sont rassemblées, lavées à l'eau, séchées sur sulfate de magnésium et évaporées à sec. Le résidu est cristallisé dans le méthanol et donne 5,9 g de produit.
Rdt : 70%

U.V. (MeOH)$\lambda_{max}$: 220, 284 nm

S.M. m/e (%) : 302 (36)$M^+$, 270 (100), 214 ( 66), 183 (17), 154 (21)

I.R. (KBr) : 3460, 3000, 2450, 1730, 1690, 1480, 1460, 1440, 1410 $cm^{-1}$

b) A une suspension de 1,38 g (0,0364 m) d'hydrure de lithium dans 100 ml de tétrahydrofuranne, on ajoute goutte à goutte sous argon une solution de 5,5 g (0,0182 m) d'azépinoindole préparé en a) dissous dans 100 ml de tétrahydrofuranne. Un traitement analogue à celui décrit dans l'exemple 1 permet d'isoler 4,1 g de résidu organique qui, par cristallisation dans le méthanol, donne 3,03 g de produit.
Rdt : 72,3%

U.V. (MeOH) $\lambda_{max}$: 290, 283, 223 nm

S.M. m/e (%) : 230 $M^+$

I.R. (KBr) : 3460, 3400, 3200, 3000, 2900, 1460, 1335, 1123 $cm^{-1}$

R.M.N. ($CDCl_3$)$\delta$: 8,03 (bs, 1H), 7,50-6,76 (m, 4H), 4,61 (m, 1H), 3,90 (m, 2H), 3,20-2,32 (m, 10H) dont 2,43 (s, 3H).

0203902

Exemple 3 : 1,2,3,4,5,6-hexahydro-3-éthyl-5-hydroxyméthyl-azépino [4,5-b]indole. (I : $R_1 = C_2H_5$; $R_2$, $R_3$, $R_4$ = H; n = 1)

a) 1,2,3,4,5,6-hexahydro-3-acétyl-5-carbométhoxy-azépino [4,5-b] indole. (IV : $R_1 = COCH_3$; $R_3$, $R_4$ = H)

6 g (0,024 m) de 5-carbométhoxy azepino [4,5-b] indole sont mis en suspension dans 350 ml d'acétate d'ethyle. Après addition de 350 ml de soude caustique 2 M, on ajoute sous forte agitation 2,88 g (0,0368 m) de chlorure d'acétyle en solution dans 30 ml d'acétate d'éthyle.
Un traitement analogue à celui décrit dans l'exemple 2 a) permet d'isoler 6,1 g de matière organique qui, par cristallisation dans l'acétate d'éthyle, donne 5,7 g de produit.
Rdt : 80,8 %

b) Suivant la procédure décrite dans l'exemple 1, on obtient à partir de 9,5 g de produit préparé en a, 3,5 g de produit.
Rdt : 43%

Fus : 144,5°C.

U.V. (MeOH)$\lambda_{max}$: 291, 283 nm

S.M. m/e (%) : 244 ($M^+$ 51), 229 (25), 186 (14), 157 (100), 144 (14), 130 (11), 71 (51).

I.R. (KBr) : 3237, 3063, 2910, 2840, 1465, 1344, 1203, 1067 $cm^{-1}$

R.M.N. ($CDCl_3$)$\delta$: 8,40 (bs, 1H), 7,56-6,83 (m, 4H), 5,60 (bs, 1H), 4,03-3,56 (m, 2H), 3,33-2,26 (m, 9H) dont 1,10 (tr, 3H).

Exemple 4 : 1,2,3,4,5,6-hexahydro-3-propyl-5-hydroxyméthyl azépino [4,5-b] indole. (I : $R_1 = C_3H_7$; $R_2$, $R_3$, $R_4$ = H; n = 1).

a) 1,2,3,4,5,6-hexahydro-3-propyl-5-carbométhoxy-azépino [ 4,5-b ] indole (IV : $R_1 = C_3H_7$; $R_3$, $R_4$ = H)

Une solution de 6,5 g (0,026 m) de 5-carbométhoxy-azepino [4,5-b ] indole et de 2 g (0,034 m) de propionaldéhyde dans 130 ml de méthanol est agitée à température ambiante pendant 4 heures. Le méthanol et l'excès d'aldéhyde sont ensuite distillés sous vide. Le résidu est dissous dans 130 ml de méthanol. Après addition de 3,25 g (0,026 m) d'acide benzoïque et de 3,25 g (0,0157 m) de cyanoborohydrure de sodium, le mélange est agité 10 heures à température ambiante. Après évaporation du solvant, le résidu est repris dans 200 ml de chlorure de méthylène, 185 ml d'eau et 65 ml d'une solution aqueuse saturée en carbonate de potassium. La phase organique est décantée et la phase aqueuse est à nouveau extraite 2 fois par 250 ml de chlorure de méthylène. Les phases organiques sont rassemplées, lavées par une solution aqueuse saturée en sel et séchées sur sulfate de sodium. Après filtration et évaporation, on obtient 7,87 g de résidu organique qui, par cristallisation dans un mélange hexane-éther donne 4,8 g de produit.
Rdt : 63%

Fus : 86-87°C

U.V. (MeOH) $\lambda_{max}$: 291, 284 nm

S.M. m/e (%) : 286 ($M^+$ 47,5), 257 (49,5), 215 (20), 202 (31), 183 (14), 169 (8), 156 (45,5), 143 (11,5), 128 (13), 84 (100)

I.R. ($CCl_4$) : 3450, 2960, 2820, 1731, 1465, 1439, 1166 $cm^{-1}$

R.M.N. ($CDCl_3$)δ: 8,26 (bs, 1H); 7,53-6,83 (m, 4H); 4,03 (dd, 4H); 3,73 (s, 3H); 3,40-2,80 (m, 6H), 2,60 (tr, 2H); 1,53 (m, 2H); 0,90 (tr, 3H).

b) Suivant la procédure décrite dans l'exemple 1, on obtient le produit avec un rendement de 62%.

Fus : 106-109°C

U.V. (MeOH) $\lambda_{max}$: 225, 282, 290 nm

S.M. m/e (%) : 258 ($M^+$ 89), 229 (69); 186 (58); 174 (36); 157 (100), 144 (20); 129 (17); 115 (8); 84 (38).

I.R. ($CHCl_3$) : 3625, 3474, 3023, 2970, 2840, 1520, 1465, 1340, 1230, 1049 $cm^{-1}$.

R.M.N. ($CDCl_3$)δ: 7,53-6,86 (m, 4H); 5,43 (bs, 1H); 3,76 (m, 2H); 3,23-2,20 (m, 9H), 1,90-1,23 (m, 2H); 0,93 (tr, 3H); 8,50 (bs, 1H).

Exemple 5 : 1,2,3,4,5,6-hexahydro-3-benzyl-5-hydroxyméthyl-azépino [4,5-b]indole. (I : $R_1 = CH_2-C_6H_5$; $R_2$, $R_3$, $R_4$ = H; n = 1).

Une solution de 7 g (0,0324 m) de 5-hydroxyméthylazépino [4,5-b] indole, de 5,02 g (0,0388 m) de diisopropyléthylamine, de 6,67 g (0,0388 m) de bromure de benzyle dans 150 ml de chloroforme sont chauffés au reflux pendant 10 heures. Le mélange réactionnel est ensuite noyé à l'eau et alcalinisé par une solution aqueuse saturée de carbonate de potassium. La phase organique est décantée et la phase aqueuse est extraite à nouveau par deux fois 100 ml de chloroforme. Les phases organiques sont rassemblées, lavées à l'eau, séchées sur sulfate de magnésium, filtrées et évaporées à sec sous vide. Le résidu, recristallisé dans l'éther, donne 7,7 g de produit.
Rdt : 77,5%.

Fus. : 95°C

R.M.N ($CDCl_3$)δ: 8,30 (bs, 1H); 7,66-6,90 (m, 9H); 4,43 (bs, 1H); 3,93-3,50 (m, 4H); 3,33-2,26 (m, 7H).

Exemple 6 : 1,2,3,4,5,6-hexahydro-3-méthyl-5-benzoyloxyméthyl-azépino [4,5-b ]indole (I : $R_1 = CH_3$; $R_2 = CO-C_6H_5$; $R_3$, $R_4$ = H; n = 1).

A une solution de 4,44 g (0,0193 m) de 3-méthyl-5-hydroxyméthyl-azépino [4,5-b] indole et de 1,84 g (0,023 m) de pyridine dans 150 ml de chloroforme, on ajoute 3,27 g (0,023 m) de chlorure de benzoyle dans 50 ml de chloroforme. Après une nuit d'agitation à la température ambiante, le mélange réactionnel est noyé à l'eau et alcalinisé par une solution aqueuse saturée de carbonate de potassium. La phase organique est ensuite traitée comme dans l'exemple 5. Le résidu obtenu est cristallisé dans l'éthanol et donne 4,1 g de produit.
Rdt : 63,6 %

Fus : 124°C

U.V. (MeOH) $\lambda_{max}$: 227, 281, 292 nm

S.M. m/e (%) : 334 ($M^+$ 92), 291 (6); 278 (17); 213 (75); 197 (10), 178 (59); 170 (100); 156 (49); 104 (50).

I.R. (KBr) : 3400, 2940, 2900, 2810, 1705, 1463, 1452, 1396, 1319, 1280, 1139 $cm^{-1}$.

R.M.N. ($CDCl_3$) $\delta$:8,23-7,76 (m, 3H); 7,63-6,90 (m, 7H), 4,83-4,46 (m, 2H); 3,58-2,20 (m, 10H) dont 2.50 (s, 3H).

0203902

Exemple 7 : 1,2,3,4,5,6-hexahydro-3-éthyl-5-benzoyloxyméthyl-azépino [4,5-b] indole (I : $R_1 = C_2H_5$; $R_2 = CO-C_6H_5$; $R_3$, $R_4$ = H; n = 1).

Suivant la procédure décrite dans l'exemple 6, le 3-éthyl-5-hydroxy méthyl-azépino [4,5-b] indole, donne le produit.
Rdt : 75%

Fus : 115-117°C

U.V. (MeOH) $\lambda_{max}$: 226, 281, 292 nm

S.M. m/e (%) : 348 ($M^+$ 27), 278 (8); 226 (31); 211 (6); 192 (30), 170 (100); 156 (47); 105 (59).

I.R. (KBr) : 3385, 3060, 2980, 2830, 1752, 1455, 1393, 1327, 1280, 1130, 717 $cm^{-1}$.

R.M.N. ($CDCl_3$)δ:8,10-7,66 (m, 3H); 7,50-6,80 (m, 7H), 4,76-4,46 (m, 2H); 3,46-2,43 (m, 9H), 1,10 (tr, 3H).

0203902

Exemple 8 : 1,2,3,4,5,6-hexahydro-5-hydroxyméthyl-6-méthyl-azépino [4,5-b ] indole (I : $R_1$, $R_2$, $R_4$ = H; $R_3$ = $CH_3$; n = 1).

Suivant la procédure décrite dans l'exemple 1, le 5-carbométhoxy-6-méthyl azépino[4,5-b] indole, donne par réduction le produit.
Rdt : 78%.

Fus : 124-126°C

U.V. (MeOH) $\lambda_{max}$: 226, 285, 292 nm

S.M. m/e (%) : 230 ($M^+$ 34), 212 (30); 200 (8); 188 (44); 170 (100); 144 (12); 128 (6); 115 (7); 91 (3).

I.R. (KBr) : 3150, 2920, 1611, 1470, 1435, 1358, 1240, 1191, 1062, 745 $cm^{-1}$.

R.M.N. ($CDCl_3$)δ: 7,53-6,83 (m, 4H); 3,91 (dd, 2H); 3,58 (s, 3H); 3,51 (s, 2H); 3,45-2,56 (m, 7H).

Exemple 9 : 1,2,3,4,5,6-hexahydro-3,6-diméthyl-5-hydroxyméthyl-azépino [ 4,5-b ] indole (I : $R_1$, $R_3$ = $CH_3$; $R_2$, $R_4$ = H; n = 1).

a) 1,2,3,4,5,6-hexahydro-3,5-dicarbométhoxy-6-méthyl-azépino [4,5-b ] indole (IV : $R_1$ = $COOCH_3$; $R_3$ = $CH_3$; $R_4$ = H).

Suivant la procédure décrite dans l'exemple 2a), 12 g (0,045 m) de 5-carbométhoxy-6-méthyl azépino [ 4,5-b ] indole donne, par condensation avec 5,52 g (0,0585 m) de chloroformiate de méthyle, 12 g de produit. Rdt : 84,4%

Fus : 107-110°C

U.V. (MeOH) $\lambda_{max}$: 226, 286 nm

S.M. m/e (%) : 316 ($M^+$ 89), 284 (100); 269 (30,7); 257 (72); 241 (25); 229 (60); 228 (87); 216 (56,7); 209 (15); 198 (42,5); 197 (54); 170 (75,7); 154 (23).

I.R. (KBr) : 3460, 3000, 2950, 2900, 1780, 1700, 1690, 1470, 1440, 1410, 1400 $cm^{-1}$.

b) Suivant la procédure décrite dans l'exemple 2b, 11 g (0,0348 m) d'azepino indole obtenu en a) sont traités par 2,642 g (0,0696 m) d'hydrure de lithium aluminium et donnent 9,5 g de matière organique qui, par cristallisation dans l'éthanol, donne 7,3 g de produit. Rdt : 86%.

Fus : 136°C

U.V. (MeOH) $\lambda_{max}$: 228, 285, 293 nm

S.M. m/e (%) : 244 ($M^+$ 81), 214 (37); 200 (9); 186 (79); 171 (100) 158 (28); 144 (15); 122 (18); 115 (9); 74 (36); 58 (70).

I.R. (KBr) : 3140, 2920, 2815, 1465, 1612, 1568, 1323, 1240, 1063 1057, 985, 743 $cm^{-1}$.

R.M.N. ($CDCl_3$)δ:7,56-6,90 (m, 4H); 5,15 (bs, 1H); 3,93 (dd, 2H) 3,56 (s, 3H); 3,23-2,83 (m, 5H), 2,58 (d, 1H) 2,43 (s, 3H); 2,26 (m, 1H).

Exemple 10 : 1,2,3,4,5,6-hexahydro-3-éthyl-5-hydroxyméthyl-6-m azépino[4,5-b]indole (I : $R_1 = C_2H_5$; $R_2$, $R_4$ = H; $R_3 = CH_3$; n = 1)

a) 1,2,3,4,5,6-hexahydro-3-acetyl-5-carbométhoxy-6-méthyl-azépino [ indole (IV : $R_1 = COCH_3$; $R_3 = CH_3$; $R_4$ = H).

Suivant la procédure décrite dans l'exemple 3a), on obtient à part 2,58 g de 5-carbométhoxy-6-méthyl azépino[4,5-b]indole, 2,3 g de pı Rdt : 76,6 %.

Fus : 131-132°C

U.V. (MeOH) $\lambda_{max}$: 226, 286 nm

S.M. m/e (%) : 300 ($M^+$ 19,21); 268 (36,5); 257 (3,9); 241 (16,4) (40); 225 (20); 216 (33); 209 (25); 184 (24); 170 157 (14); 41 (100).

I.R. (KBr) : 3460, 3040, 3000, 2960, 1740, 1640, 1450, 1180 $cm^{-1}$

b) Suivant la procédure décrite dans l'exemple 2b), on obtient, à p de 2 g de produit décrit en a), 1 g de produit Rdt : 69,7 %

Fus : 70 - 72°C

U.V. (MeOH) $\lambda_{max}$: 228, 285, 292 nm

S.M. m/e (%) : 258 ($M^+$ 47); 228 (23); 212 (16); 188 (30); 186 184 (20); 171 (100); 158 (23); 144 (17); 129 (22 (16); 72 (37); 41 (30).

I.R. (KBr) : 3160, 3059, 2920, 2830, 1606, 1471, 1403, 1240, 1192, 1152, 1059, 951, 919, 738 $cm^{-1}$.

R.M.N. ($CDCl_3$)δ: 7,53-6,80 (m, 4H); 5,20 (bs, 1H); 4,23-3,76 (m, 2H); 3,53 (s, 3H); 3,33-2,30 (m, 9H) dont 2,56 (q, 2H); 1,13 (tr, 3H).

Exemple 11 : 1,2,3,4,5,6-hexahydro-3-méthyl-5-hydroxyméthyl-6-éthyl-azépino [4,5-b] indole (I : $R_1 = CH_3$; $R_2$, $R_4$ = H; $R_3 = C_2H_5$; n = 1).

Suivant la procédure décrite dans l'exemple 2 à partir de 3-5 dicarbo-méthoxy-6-éthyl-azépino[4,5-b]indole, on obtient le produit.

Fus : 129°C

U.V. (MeOH) $\lambda_{max}$: 228, 285, 290 nm

S.M. m/e (%) : 258 (56 $M^+$); 200 (49); 185 (100); 184 (61); 129 (13); 74 (10); 58 (22).

I.R. (KBr) : 3160, 2980, 2920, 2820, 1460, 1430, 1380, 1350, 1320, 1290, 1280, 1240 $cm^{-1}$.

Exemple 12 : 1,2,3,4,5,6-hexahydro-3,6-diéthyl-5-hydroxyméthyl-azépino [4,5-b]indole (I : $R_1$, $R_3$ = $C_2H_5$; $R_2$, $R_4$ = H; n = 1)

a) 1,2,3,4,5,6-hexahydro-3-acétyl-5-carbométhoxy-6-éthyl-azépino [ 4,5-b] indole (IV : $R_1$ = $COCH_3$; $R_3$ = $C_2H_5$; $R_4$ = H).

Suivant la procédure décrite dans l'exemple 3a), on obtient à partir de 7 g (0,0257 m) de 5-carbométhoxy-6-éthyl azépino[4,5-b]indole, 4.5 g de produit.

Rdt : 56 %

S.M. m/e (%) : 314 (67 $M^+$); 282 (93); 255 (27); 243 (36); 242 (100); 239 (36); 230 (63); 223 (28); 211 (20); 184 (46); 182 (30); 168 (23); 154 (21); 43 (30).

b) Suivant la procédure décrite dans l'exemple 2b), on obtient à partir de 3 g de produit décrit en a), 2.1 g de produit.

Rdt : 81 %

Fus : 103°C

U.V. (MeOH) $\lambda_{max}$: 229, 285, 293 nm

S.M. m/e (%) : 272 (59 $M^+$); 242 (14); 214 (10); 200 (71); 185 (100); 184 (77); 172 (16); 158 (13); 136 (16); 88 (21); 72 (53); 42 (29).

I.R. (KBr) : 3180, 2980, 2840, 1470, 1370, 1350, 1330, 1270, 1230 $cm^{-1}$.

Exemple 13 : 1,2,3,4,5,6-hexahydro-3-diméthylaminoéthyl-5-hydroxyméthyl azépino[ 4,5-b] indole

(I : $R_1 = CH_2-CH_2-N<^{CH_3}_{CH_3}$ ; $R_2$, $R_3$, $R_4$ = H; n = 1)

Une solution de 7 g (0,0324 m) de 5-hydroxyméthyl azépino[4,5-b] indole de 10,04 g (0,0777 m) de diisopropyléthylamine, de 5.6 g (0,0388 m) de 1-chloro 2 diméthylamino éthane chlorhydrate dans 150 ml de chloroforme sont chauffés au reflux pendant 15 heures. Le mélange réactionnel est ensuite noyé à l'eau et alcalinisé par une solution aqueuse saturée de carbonate de potassium. La phase organique est décantée et la phase aqueuse est extraite à nouveau par deux fois 100 ml de chloroforme. Les phases organiques sont rassemblées, lavées à l'eau, séchées sur sulfate de magnésium, filtrées et évaporées à sec sous vide. Le résidu est cristallisé dans l'acétate d'éthyle et donne 8,1 g de produit.

Rdt : 87 %

Fus : 182-184°C

U.V. (MeOH) $\lambda_{max}$: 226, 282, 290 nm

S.M. m/e (%) : 287 (8 $M^+$); 257 (12); 230 (14); 229 (89); 199 (20); 186 (100); 170 (26); 168 (21); 156 (31); 144 (16); 101 (53); 58 (88).

I.R. (KBr) : 3240, 3060, 2930, 2820, 1621, 1588, 1419, 1368, 1191, 1139, 1047, 917 $cm^{-1}$.

R.M.N. ($CDCl_3$ + $CD_3$ OD)δ: 2,30 (s, 6H); 3,06-2,41 (m, 8H); 4,16-3,05 (m,5H); 7,53-6,80 (m, 4H).

Exemple 14 : 1,2,3,4,5,6-hexahydro-3-diéthylaminoéthyl-5-hydroxyméthyl-azépino [4,5-b]indole

($I$ : $R_1 = CH_2\text{-}CH_2\text{-}N\langle^{C_2H_5}_{C_2H_5}$ ; $R_2$, $R_3$, $R_4$ = H; n = 1)

a) 1,2,3,4,5,6-hexahydro-3-diéthylaminoacétyl-5-carbométhoxyazépino [4,5-b]indole (VI : R' = $C_2H_5$; $R_3$, $R_4$ = H; m = 1)

6 g (0,0245 m) de 5-carbométhoxyazépino [4,5-b] indole sont mis en suspension dans 350 ml d'acétate d'éthyle. Après addition de 350 ml de soude caustique 2M, on ajoute sous forte agitation, 3.25 g (0,0288 m) de chlorure de chloracétyle en solution dans 30 ml d'acétate d'éthyle. Après 10 minutes d'agitation, la réaction est terminée. On décante la phase organique et on extrait deux fois la phase aqueuse par 50 ml d'acétate d'éthyle. Les phases organiques sont rassemblées, lavées à l'eau, séchées sur sulfate de sodium et évaporées à sec sous vide. Le résidu est alors repris dans 400 ml de tétrahydrofuranne. On ajoute ensuite 3,87 g (0,053 m) de diéthylamine et on agite pendant 12 heures à température ambiante. Lorsque la réaction est terminée, on évapore le milieu réactionnel à sec sous vide et on dissout le résidu dans 150 ml de chlorure de méthylène. Le chlorure de méthylène est ensuite lavé à l'eau, séché sur sulfate de sodium et évaporé à sec sous vide. Le résidu est dissous dans l'acétone et isolé sous forme de chlorhydrate. Poids : 7,3 g. Rdt : 75,5 %

U.V. (MeOH) $\lambda_{max}$: 222, 289, 292 nm

S.M. m/e (%) : 357 (51 $M^+$); 214 (5); 169 (12); 143 (7); 86 (100);

I.R. (KBr) : 3200, 2968, 2840, 1730, 1630, 1460 $cm^{-1}$.

R.M.N. ($CDCl_3$)δ : 7,53-6,76 (m, 4H); 4,46-3,50 (m, 8H) dont 3,70 (s, 2H) et 3,63 (s, 3H), 3,43-2,83 (m, 4H); 2,46 (q, 4H); 0,96 (tr, 6H).

b) A une suspension de 1,3 g (0,0343 m) d'hydrure de lithium aluminium dans 100 ml de tétrahydrofuranne anhydre est ajouté goutte à goutte sous argon, une solution de 4 g (0,0112 m) de produit préparé en a), dissous dans 100 ml de tétrahydrofuranne. Un traitement analogue à celui décrit dans l'exemple 1 permet d'isoler 3,1 g de produit par cristallisation dans l'acétonitrile.

Rdt : 88 %

Fus. : 163°C

U.V. (MeOH) $\lambda_{max}$: 225, 282, 290 nm

S.M. m/e (%) : 315 (0,3 $M^+$); 229 (2); 156 (2); 100 (3); 86 (100); 72 (8); 70 (3); 58 (32); 54 (10); 44 (11); 42 (16).

I.R. (KBr) : 3230, 3060, 2980, 2880, 2820, 1620, 1589, 1469, 1373, 1196, 1139, 1042, 919, 731 $cm^{-1}$.

R.M.N. ($CDCl_3$ + $CD_3OD$) δ:

7,45-6,80 (m, 4H); 4 (dd, 1H); 3,60 (dd, 1H), 3,40-3,08 (m, 2H); 3,06-2,26 (m, 10H) dont 2,56 (q, 4H); 1,06 (tr, 6H).

<u>Exemple 15 : 1,2,3,4,5,6-hexahydro-3-morpholinoéthyl-5-hydroxyméthyl-azépino[4,5-b] indole</u> (I : $R_1$ = $CH_2$-$CH_2$-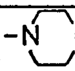; $R_2$, $R_3$, $R_4$ = H; n = 1).

a) 1,2,3,4,5,6-hexahydro-3-morpholinoacétyl-5-carbométhoxy-azépino [4,5-b]indole (VI : $N<^{R'}_{R'}$ = 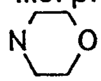; $R_3$, $R_4$ = H; m = 1)

Par le procédé décrit dans l'exemple 14 a), on obtient le produit sous forme de chlorhydrate.

U.V. (MeOH) $\lambda_{max}$: 223, 284, 292 nm

S.M. m/e (%) : 371 (35 $M^+$); 313 (3); 227 (11); 214 (7); 183 (5); 169 (10); 156 (10); 100 (100).

I.R. (KBr) : 3260, 2950; 2860, 1730, 1635, 1460, 1115, 1012 $cm^{-1}$.

R.M.N. ($CDCl_3$)$\delta$:8,93 (m, 1H); 7,53-6,83 (m, 4H); 4,26-3,96 (m, 3H); 3,96-3,53 (m, 9H) dont 3,61 (s, 3H); 3,32-2,83 (m, 4H) dont 3,15 (s, 2H); 2,60-2,20 (m, 4H).

b) 1,2,3,4,5,6-hexahydro-3-morpholinoacetamido-5-carbomethoxy-azépino [4,5-b]indole (VII : $N<^{R'}_{R'}$ = 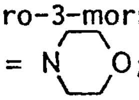; $R_3$, $R_4$ = H; m = 1)

Une solution de 6 g (0,0245 m) de 5-carbométhoxy azépino [4,5-b] indole, de 3,81 (0,0295 m) de diisopropyléthylamine, de 4,82 g (0,0295 m) de chloroacétylmorpholine dans 150 ml de chloroforme sont chauffés au reflux pendant 5 heures. Le mélange réactionnel est ensuite traité comme dans l'exemple 5 pour donner 7,1 g de produit.
Rdt : 77,8 %.
Le produit est cristallisé sous forme de méthane sulfonate.

U.V. (MeOH) $\lambda_{max}$: 225, 284, 292 nm

I.R. (KBr) : 3271, 2963; 2900, 2858, 1723, 1630, 1463, 1441, 1274, 1120 $cm^{-1}$.

R.M.N. ($CDCl_3$)δ:8,40 (bs, 1H); 7,56-6,93 (m, 4H); 3,96 (dd, 1H); 3,66 (s, 3H); 3,63 (m, 8H); 3,48 (s, 2H); 3,36 (d, 1H), 3,16 (d, 1H); 2,93 (m, 4H).

c) La réduction des produits décrite en a) et en b) par l'hydrure-de lithium aluminium suivant le procédé décrit dans l'exemple 1 permet d'obtenir le produit.

Fus. : 268°C

U.V. (MeOH)$\lambda_{max}$: 226, 283, 291 nm

I.R. (KBr) : 3250, 2900; 2800, 1468, 1359, 1310, 1150, 1114, 1043, 928 $cm^{-1}$.

R.M.N. ($CDCl_3$)δ:8,80 (m, 1H); 7,56-6,73 (m, 4H); 4,16 (m, 1H); 4,00-3,23 (m, 7H); 3,23-2,00 (m, 14H).

<u>Exemple 16 : 1,2,3,4,5,6-hexahydro-3-pipéridinoéthyl-5-hydroxyméthyl-azépino [4,5-b] indole</u> (I : $R_1 = CH_2-CH_2-$N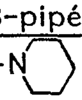 ; $R_2$, $R_3$, $R_4$ = H; n = 1)

a) 1,2,3,4,5,6-hexahydro-3-pipéridinoacetyl-5-carbométhoxy-azépino [ 4,5-b ] indole (VI : $N<^{R'}_{R'}$ = N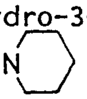 ; $R_3$, $R_4$ = H; m = 1)

Par le procédé décrit dans l'exemple 14 a), on obtient le produit sous forme de base.

Fus. : 149°C

U.V. (MeOH)$\lambda_{max}$: 223, 284, 292 nm

S.M. m/e (%) : 369 (32 $M^+$); 310 (1,2); 227 (1,94); 214 (2,46); 183 (2,29); 169 (3,62); 155 (5,23); 98 (100); 84 (14).

I.R. (KBr) : 3280, 2940, 1740, 1630, 1460, 1460, 1450, 1430, 1380, 1310 $cm^{-1}$.

b) La réduction du produit décrit en a) suivant le procédé décrit dans l'exemple 1 permet d'obtenir le produit.

Fus.: 174°C

U.V. (MeOH)$\lambda_{max}$: 226, 282, 290 nm

S.M. m/e (%) : 327 (1 $M^+$); 229 (6); 186 (8); 170 (4); 156 (7); 141 (22); 129 (13); 98 (100); 86 (17); 70 (7); 58 (10); 55 (14).

I.R. ($CHCl_3$) : 2950, 2825, 1458, 1481, 1355, 1339, 1312, 1143, 1111, 1038, 977, 924 $cm^{-1}$.

R.M.N. ($CDCl_3$)$\delta$:9,00 (bs, 1H); 7,56-6,76 (m, 4H); 4,03 (m, 1H); 3,80-3,26 (m, 2H); 3,13-1,93 (m, 15H), 1,90-1,20 (m, 6H)

Exemple 17 : 1,2,3,4,5,6-hexahydro-3-pyrrolidinoéthyl-5-hydroxyméthyl-azépino[4,5-b ] indole (I : $R_1$ = $CH_2$-$CH_2$-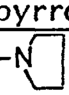 ; $R_2$, $R_3$, $R_4$ = H; n = 1)

a) 1,2,3,4,5,6-hexahydro--3-pyrrolidinoacétyl-5-carbométhoxyazépino [4,5-b] indole (VI : 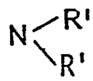 = 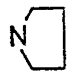 ; $R_3$, $R_4$ = H; m = 1)

Par le procédé décrit dans l'exemple 14a), on obtient le produit sous forme de base.

Fus.: 166°C

U.V. (MeOH) $\lambda_{max}$: 283, 292 nm

S.M. m/e (%) : 355 (22 $M^+$); 183 (3); 129 (3); 85 (16); 84 (100); 82 (4); 55 (15); 42 (34).

I.R. (KBr) : 3400, 3340, 2960, 2900, 1735, 1650, 1620, 1610, 1460, 1430, 1380 $cm^{-1}$.

b) 1,2,3,4,5,6-hexahydro-3-pyrrolidinoacétamido-5-carbométhoxy-azépino [4,5-b]indole (VII : 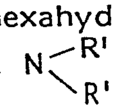 = 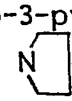 ; $R_3$, $R_4$ = H; m = 1)

Par le procédé décrit dans l'exemple 15b), on obtient le produit sous forme de base

Fus.: 166-168,5°C

U.V. (MeOH) $\lambda_{max}$: 225, 285, 292 nm

S.M. m/e (%) : 355 (34 $M^+$); 343 (8); 324 (3); 296 (2); 257 (52); 227 (20); 215 (58); 197 (14); 183 (10); 168 (13); 156 (30); 141 (100)

I.R. (KBr) : 3250, 2957, 2887, 1732, 1630, 1462, 1340, 1320, 1232, 1201, 911 $cm^{-1}$.

R.M.N. ($CDCl_3$)δ:8,6 (m, 1H); 7,56-6,80 (m, 4H); 3,93 (dd, 1H); 3,66 (s, 3H); 3,60-3,16 (m, 8H); 2,93 (m, 4H); 1,86 (m, 4H).

c) La réduction des produits décrits en a) et b) par l'hydrure de lithium aluminium suivant le procédé décrit dans l'exemple 1 permet d'obtenir le produit.

Fus : 178,2°C

U.V. (MeOH)$\lambda_{max}$: 226, 284, 291 nm

S.M. m/e (%) : 313 (5 $M^+$); 283 (10); 289 (73); 199 (7); 186 (40); 170 (10); 156 (11); 127 (46); 115 (18); 84 (100).

Exemple 18 : 1,2,3,4,5,6-hexahydro-3-diméthylaminoéthyl-5-hydroxyméthyl -6-méthyl-azépino [ 4,5-b ] indole

$$(I : R_1 = CH_2\text{-}CH_2\text{-}N\begin{matrix}CH_3\\CH_3\end{matrix};\ R_2,\ R_4 = H;\ R_3 = CH_3;\ n=1)$$

Une solution de 5 g (0,0217 m) de 5-hydroxyméthyl-6-méthyl-azépino [4,5-b] indole, de 6,74 g (0,0521 m) de diisopropyléthylamine, de 3,756 g (0,0260 m) de 1-chloro-2-diméthylamino éthane chlorhydrate dans 150 ml de chloroforme sont chauffés au reflux pendant 20 heures. Le mélange réactionnel est ensuite traité comme dans l'exemple 5. Le résidu est repris dans l'acétonitrile et donne, par cristallisation, 5,6 g de produit.
Rdt : 85,4%

Fus.: 141°C

U.V. (MeOH) $\lambda_{max}$: 228, 285, 294 nm

S.M. m/e (%) : 301 (12 $M^+$).

I.R. (KBr) : 3060, 2930, 2820, 1606, 1561, 1473, 1387, 1320, 1259, 1149, 1137, 1013, 1007, 742 $cm^{-1}$.

R.M.N. ($CDCl_3$) δ:7,50-6,76 (m, 4H); 3,90 (m, 1H); 3,60 (s, 3H); 3,66-1,96 (m, 16H); 2,30 (s, 3H).

<u>Exemple 19 : 1,2,3,4,5,6-hexahydro-3-pyrrolidinoéthyl-5-hydroxyméthyl-6-méthyl azépino [4,5-b] indole</u>

(I : $R_1 = CH_2-CH_2-$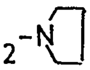 ; $R_2$, $R_4$ = H; $R_3 = CH_3$; n = 1)

Suivant le procédé décrit dans l'exemple 18, la condensation de 1-chloro-2-pyrrolidino-éthane chlorhydrate avec le 5-hydroxyméthyl-6-méthyl-azépino [ 4,5-b ] indole, donne le produit.

Fus.: 117°C

U.V. (MeOH) $\lambda_{max}$: 227, 285, 293 nm

S.M. m/e (%) : 327 (1 $M^+$); 297 (35); 243 (100); 213 (11); 200 (50); 183 (24); 182 (23); 170 (18); 127 (46); 84 (93); 42 (17)

I.R. (KBr) : 3180, 2990, 2800, 1470, 1450, 1370, 1350, 1330, 1240, 1190, 1140, 1120, 1060, 1050 $cm^{-1}$.

Exemple 20 : 1,2,3,4,5,6-hexahydro-3-diéthylaminoéthyl-5-hydroxyméthyl-6-méthyl-azépino [4,5-b] indole

(I : $R_1 = CH_2-CH_2-N<\begin{matrix}C_2H_5\\C_2H_5\end{matrix}$; $R_2$, $R_4$=H; $R_3$=$CH_3$; n=1)

Suivant le procédé décrit dans l'exemple 18, la condensation du 1-chloro-2 diéthylamino éthane chlorhydrate avec le 5-hydroxyméthyl-6-méthyl-azépino[4,5-b] indole donne le produit.

Fus.: 97-98°C

U.V. (MeOH) $\lambda_{max}$: 228, 285 nm

S.M. m/e (%) : 329 (4 $M^+$); 300 (26); 299 (81); 244 (49); 243 (100); 213 (13); 200 (83); 184 (35); 183 (27); 170 (35); 129 (39); 86 (93)

I.R. (KBr) : 3140, 2960, 2840, 1460, 1360, 1320, 1270, 1250, 1240, 1190, 1140, 1050, 1000 $cm^{-1}$.

Exemple 21 : 1,2,3,4,5,6-hexahydro-3-morpholinoéthyl-5-hydroxyméthyl-6-méthyl-azépino [4,5-b] indole

(I : $R_1$ = $CH_2$-$CH_2$-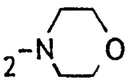; $R_2$, $R_4$=H; $R_3$=$CH_3$; n=1)

Suivant le procédé décrit dans l'exemple 18, la condensation du 1-chloro-2-morpholinoéthane chlorhydrate avec le 5-hydroxyméthyl-6-méthyl azépino [4,5-b]indole donne le produit.

Fus.: 120°C

U.V. (MeOH) $\lambda_{max}$: 228, 285, 292 nm

S.M. m/e (%) : 343 (2 $M^+$); 313 (34); 244 (100); 200 (67); 184 (30); 170 (26); 158 (10); 143 (23); 100 (26).

I.R. (KBr) : 3220, 2960, 2940, 2900, 1470, 1390, 1370, 1350, 1320, 1300, 1280, 1250, 1190, 1150, 1120, 1050, 1030 $cm^{-1}$.

Exemple 22 : 1,2,3,4,5,6-hexahydro-3-éthyl-5-hydroxyéthyl-azépino [4,5-b]indole (I : $R_1 = C_2H_5$; $R_2$, $R_3$, $R_4$ = H; n = 2)

a) 1,2,3,6 tétrahydro-3-ethyl-5-carbométhoxymethyl-azépino [4,5-b] indol-4-one.

15 g de 1-carbométhoxyméthyl, 1'-carbométhoxy, 2-éthyl, tétrahydro-1,2, 3,4 β carboline dissous dans 225 ml de toluène et 45 ml d'acide acétique sont chauffés au reflux pendant 10 heures. Le milieu réactionnel est ensuite alcalinisé par une solution de soude caustique 2M jusqu'à pH 9. La phase organique est décantée, la phase aqueuse est extraite à nouveau par deux fois 150 ml de toluène. Les phases organiques sont rassemblées, lavées à l'eau jusqu'à neutralité, séchées sur sulfate de magnésium et évaporées à sec sous vide. On obtient 8 g de résidu. Le résidu est dissous dans 150 ml d'acide acétique glacial et hydrogéné à pression atmosphérique en présence de 1 g de charbon palladié à 20%. Après 24 heures, la réaction est terminée. Le catalyseur est filtré et la solution acétique est alcalinisée jusqu'à pH 9 par une solution aqueuse de soude caustique 2M. La phase aqueuse alcaline est extraite 3 fois par 150 ml de chlorure de méthylène. Les phases organiques rassemblées sont lavées à l'eau jusqu'à neutralité, séchées sur sulfate de magnésium et évaporées à sec. Le résidu est recristallisé dans le méthanol et donne 10,8 g de produit.
Rdt : 79%.

Fus.: 153°C

U.V. (MeOH) $\lambda_{max}$: 222, 283, 290 nm

S.M. m/e (%) : 300 (100 $M^+$); 269 (41); 268 (70); 241 (56); 240 (82); 170 (87); 168 (33); 156 (89); 155 (44); 129 (25).

I.R. (KBr) : 3300, 2990, 1740, 1640, 1490, 1450, 1380, 1360, 1330, 1290, 1260, 1200, 1020 $cm^{-1}$.

b) A une suspension de 2,53 g d'hydrure de lithium aluminium dans 100 ml de tétrahydrofuranne, on ajoute goutte à goutte sous argon 10 g de produit préparé en a) dissous dans 100 ml de tétrahydrofuranne. Le milieu réactionnel est ensuite traité comme dans l'exemple 1 et on obtient 7,1 g de produit.

Rdt : 82%

Fus.: 152°C

U.V. (MeOH) $\lambda_{max}$: 224, 281, 289 nm

S.M. m/e (%) : 258 (97 $M^+$); 227 (15); 201 (37); 188 (85); 170 (83); 168 (36); 156 (100); 154 (31); 144 (30); 128 (24); 72 (63); 43 (45).

I.R. (KBr) : 3200, 2980, 2920, 2840, 2680, 1460, 1450, 1380, 1350, 1340, 1200, 1140, 1070, 1020, 1010 $cm^{-1}$.

0203902

Les composés de l'invention ont fait l'objet d'une étude pharmacologique.

1. Toxicité.

La dose létale 50 ($DL_{50}$) des composés est déterminée chez des souris de souche CD1 par méthode graphique. La $DL_{50}$ va de 40 à 2000 mg/kg par voie orale. Elle a été calculée selon la méthode de standard de LICHTFIELD et WILCOXON (1970).

2. Hypoxie hypobare.

Des souris de souche CD1 sont maintenues dans une atmosphère appauvrie en oxygène, par réalisation d'un vide partiel (190 mm de mercure correspondant à 5,25% d'oxygène).
Le temps de survie des animaux est noté. Ce temps est augmenté par les agents capables de favoriser l'oxygénation tissulaire et en particulier cérébrale.

Les composés étudiés sont administrés par voie orale, à plusieurs doses, 30 minutes avant l'essai.

Les résultats sont exprimés en pourcentage d'augmentation du temps de survie par rapport aux animaux non traités.

En portant en graphique le pourcentage de survie des animaux traités par rapport aux contrôles, en fonction du logarithme de la dose de produit administrée, on peut déterminer la "$ED_{50}$" (effective dose), c'est à dire la dose exprimée en mg/kg induisant 50% d'activité dans le test.
La $ED_{50}$ varie de 1,9 à 200 mg/kg p.o. environ.

En vue d'évaluer l'effet thérapeutique d'un produit, on compare la valeur de l'$ED_{50}$ du produit à la $LD_{50}$ du même produit, ce qui permet de définir un indice thérapeutique (T.I.)
$TI = LD_{50}$ mg/kg/$ED_{50}$ mg/kg.
L'indice thérapeutique varie de 9 à 126.

3. Hypoxie histotoxique

Des souris de souche NMRI sont injectées par voie intraveineuse avec du cyanure de potassium (KCN) à raison de 2,5 mg/kg.

Les contrôles meurent dans les 2 minutes à raison de 80% en moyenne.

On note le pourcentage de survie obtenu, après 30 minutes, dans chaque série.

Les composés étudiés sont administrés par voie orale, à différentes doses, 30 minutes avant l'injection de KCN.

Les résultats sont exprimés en pourcentage de survie pour chaque série.

Les agents capables de favoriser l'oxygénation tissulaire et en particulier cérébrale entraînent une survie des animaux et exercent donc un effet protecteur dans ce cas d'hypoxie.

En portant en graphique le pourcentage de survie des animaux traités par rapport aux contrôles, en fonction du logarithme de la dose de produit administrée, on peut déterminer la "$ED_{50}$" (effective dose) : voir §2.

La $ED_{50}$ des composés se situe dans ce test généralement entre le 5° et le 10° de leur LD50.

L'indice thérapeutique tel que défini ci-dessus au §2 varié donc de 5 à 10.

4. Hypoxie hypoxique normobare

Des souris de souche NMRI sont maintenues dans une enceinte où passe un courant d'azote à débit contrôlé durant 25 secondes environ; ensuite, l'air est ramené dans l'enceinte par passage d'air comprimé à débit contrôlé, à raison de 30 secondes environ. Ensuite, on ouvre l'enceinte et on dépose doucement les souris dans une cage.

On note pour chaque série le pourcentage de survie obtenu après 30 min.

Les contrôles meurent dans ces conditions à raison de 90 à 100%. Les agents capables de favoriser l'oxygénation tissulaire et en particulier cérébrale entraînent une survie des animaux et exercent donc un effet protecteur dans ce cas d'hypoxie.

Les composés étudiés sont administrés par voie orale, à plusieurs doses, 30 minutes avant l'essai.
Les animaux traités avec les composés étudiés présentent un taux de survie important.
En portant en graphique le pourcentage de survie des animaux traités par rapport aux contrôles, en fonction du logarithme de la dose de produit administrée, on peut déterminer la "$ED_{50}$" : voir §2.
La $ED_{50}$ varie de 6,8 à 92 mg/kg.
L'indice thérapeutique tel que défini ci-dessus au §2 varie de 15 à 80.

REVENDICATIONS

1. Dérivés de 1,2,3,4,5,6-hexahydro-5-hydroxy-alkyl-azépino(4,5-b) indole de formule :

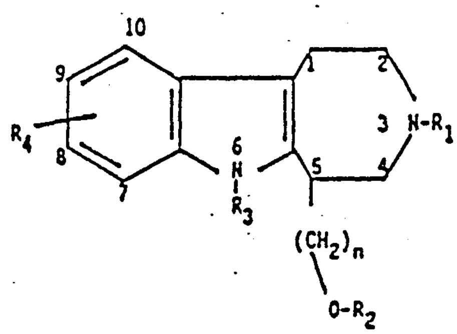

I

dans laquelle

$R_1$ représente un atome d'hydrogène, un radical alkyle, un radical alcényle, un radical benzyle, un radical alkylamino du type $-(CH_2)_m-N\langle^{R'}_{R'}$

où les groupes R' sont soit des atomes d'hydrogène, soit des radicaux alkyle ou forment ensemble avec l'atome d'azote auxquels ils sont attachés, un noyau hétérocycle du type morpholinyle, pipéridinyle, pyrrolidinyle, pipérazinyle et m est 2 ou 3,

$R_2$ représente un atome d'hydrogène, ou un radical benzoyle ou acyle

$R_3$ représente un atome d'hydrogène, un radical alkyle ou benzyle

$R_4$ représente un atome d'hydrogène ou d'halogène, un radical alkyle, un radical alcoxy, un radical trifluorométhyle, et

n est 1 ou 2 et les sels d'addition d'acides pharmaceutiquement acceptables.

2. Dérivés selon la revendication 1 caractérisé en ce qu'ils sont choisis parmi le groupe constitué par:
le 1,2,3,4,5,6-hexahydro-5-hydroxyméthyl-azépino [4,5-b] indole,
le 1,2,3,4,5,6-hexahydro-3-méthyl-5-hydroxyméthyl-azépino [4,5-b] indole,
le 1,2,3,4,5,6-hexahydro-3-éthyl-5-hydroxyméthyl-azépino [4,5-b] indole,

le 1,2,3,4,5,6-hexahydro-3-propyl-5-hydroxymethyl-azépino [4,5-b] indole,
le 1,2,3,4,5,6-hexahydro-5-hydroxymethyl-6-méthyl-azépino [4,5-b] indole
le 1,2,3,4,5,6-hexahydro-3,6-diméthyl-5-hydroxyméthyl-azépino [4,5-b] indole,
le 1,2,3,4,5,6-hexahydro-3-éthyl-5-hydroxyméthyl-6-méthyl-azépino [4,5-b] indole,
le 1,2,3,4,5,6-hexahydro-3-méthyl-5-hydroxyméthyl-6-éthyl-azépino [4,5-b] indole,
le 1,2,3,4,5,6-hexahydro-3-éthyl-5-hydroxyméthyl-azépino [4,5-b] indole.

3. Procédé pour la préparation de dérivés selon la revendication 1, dans lesquels n=1, caractérisé en ce qu'il comporte au moins la succession des étapes suivantes :

- réduction du groupement carboalkoxy d'un dérivé de 1,2,3,4,5,6-hexahydro-5-carboalkoxy-azépino(4,5-b)indole correspondant de formule générale :

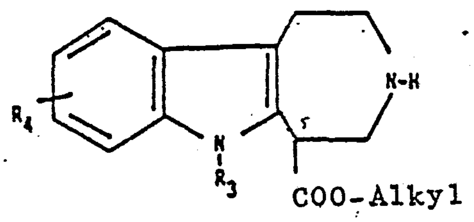

IIa

:

dans laquelle $R_3$ et $R_4$ ont la signification mentionnée à la revendication 1;

- substitution de l'azote en position 3, par les valeurs correspondantes de $R_1$ données à la revendication 1 et éventuellement

- substitution par les valeurs correspondantes de $R_2$ données à la revendication 1 de l'oxygène du groupement -OH résultant de la réduction du groupement carboalkoxy.

4. Procédé selon la revendication 3 caractérisé en ce qu'on effectue la substitution de l'azote par condensation d'un halogénure d'alkyle, de benzyle ou d'alkylamino, substitué ou non, en présence d'une amine tertiaire dans un hydrocarbure aliphatique chloré, à une température comprise entre 20°C et la température de reflux du solvant.

5. Procédé pour la préparation de dérivés selon la revendication 1, dans lesquels n=1, caractérisé en ce qu'il comporte au moins la succession des étapes suivantes :

- substitution par les valeurs correspondantes de $R_1$ données dans la revendication 1 de l'azote en position 3 du composé de formule générale

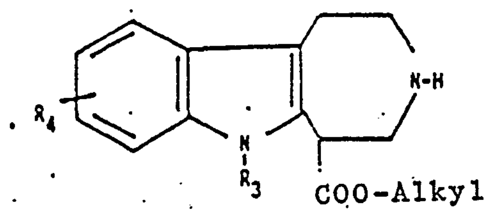

IIa

dans laquelle $R_3$ et $R_4$ ont les significations mentionnées à la revendication 1;

- réduction du groupement carboalkoxy; et éventuellement
- substitution par les valeurs correspondantes de $R_2$ données dans la revendication 1 de l'oxygène du groupement-OH obtenu après la réduction du groupement carbométhoxy.

6. Procédé selon la revendication 5 caractérisé en ce qu'on effectue la substitution de l'azote en position 3 par condensation d'un aldéhyde aliphatique ou aromatique avec le composé de départ IIa pour former les méthanoazépinoindoles de formule générale

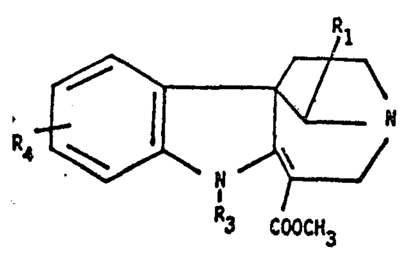

III

dans laquelle les radicaux ont les mêmes significations que dans la revendication 1, et par réduction du composé de formule III dans un solvant organique comme le méthanol ou l'éthanol, à température ordinaire, en présence de cyanoborohydrure de sodium.

7. Procédé suivant la revendication 5 caractérisé en ce qu'on effectue la substitution de l'azote en position 3, par condensation d'un halogénure d'alkyle, d'alcényle, de benzyle avec le composé IIa, en présence d'une amine tertiaire, dans un hydrocarbure aliphatique chloré.

8. Procédé suivant la revendication 5 caractérisé en ce qu'on effectue la substitution de l'azote en position 3, par condensation d'un chloroformiate d'alkyle, ou d'un chlorure d'acide dans un système binaire composé d'une solution du composé IIa dans l'acétate d'éthyle et d'une solution aqueuse de soude caustique.

9. Procédé suivant la revendication 5 caractérisé en ce qu'on effectue la substitution de l'azote en position 3, par un radical $R_1$ correspondant aux groupements alkylamino par addition d'un chlorure d'acide du type $X-(CH_2)_m-COCl$ où X représente un atome d'halogène tel que le brome ou le chlore et m=1 ou 2, à un système binaire composé d'une solution du composé IIa dans l'acétate d'éthyle et d'une solution aqueuse de soude caustique, en vue d'obtenir un produit intermédiaire de formule générale

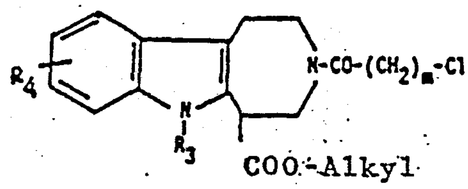

V

dans laquelle les radicaux ont les valeurs mentionnées dans la revendication 1, et par condensation du composé

V avec des amines primaires ou secondaires dans un solvant organique tel que le tétrahydrofuranne.

10. Procédé suivant la revendication 5 caractérisé en ce qu'on effectue la substitution de l'azote en position 3, par un radical $R_1$ correspondant aux groupements alkylamino par mise en contact du composé IIa avec des halogénoamides du type

$X-(CH_2)_m-CO-N\begin{matrix}R'\\R'\end{matrix}$ ou des halogènures d'alkylamine du type $X-(CH_2)_m-N\begin{matrix}R'\\R'\end{matrix}$, dans lesquels X représente un atome d'halogène, plus particulièrement de chlore ou de brome et m représente un nombre entier allant de 1 à 3, en présence d'une amine tertiaire, plus particulièrement la diisopropyléthylamine ou la triéthylamine, dans un hydrocarbure aliphatique chloré tel que le chloroforme ou le dichloroéthane, ou en présence de carbonate de potassium dans la méthylcétone.

11. Procédé pour la préparation de dérivés selon la revendication 1 dans lesquels n=2, caractérisé en ce qu'il comporte au moins la succession des étapes suivantes :

- réduction catalytique du groupement carboxyméthylène du composé répondant à la formule générale

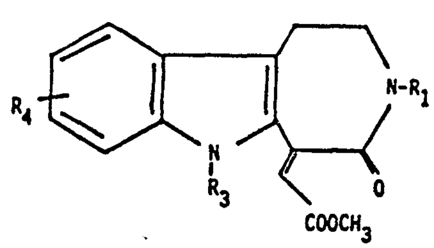

IIb

dans laquelle les radicaux ont les mêmes significations que dans la revendication 1, de manière à obtenir le composé intermédiaire de formule générale

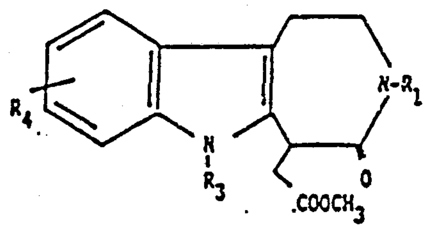

IX

dans laquelle les radicaux ont les mêmes significations que dans la revendication 1;

- réduction du groupement carbométhoxy; et éventuellement

- substitution par les valeurs correspondantes de $R_2$ données dans la revendication 1 de l'oxygène de groupement-OH en position 5, résultant de la réduction du groupement carbométhoxy.

12. Procédé selon la revendication 11 caractérisé en ce qu'on effectue la réduction catalytique du groupement carboxyméthylène du composé IIb par l'hydrogénation catalytique au moyen de charbon palladié, dans un solvant approprié, plus particulièrement l'acide acétique, à des températures comprises entre 20°C et 40°C.

13. Procédé selon l'une quelconque des revendications 3 à 12 dans lequel on effectue la réduction du groupement carbométhoxy en position 5 dans un solvant organique inerte, plus particulièrement le tétrahydrofuranne, à une température comprise entre 20°C et la température de reflux du solvant, en présence d'un agent de réduction, de préférence l'hydrure de lithium aluminium.

14. Procédé selon l'une quelconque des revendications 3 à 13 caractérisé en ce qu'on effectue la substitution par $R_2$ de l'oxygène du groupement-OH qui résulte de la réduction du groupement carbométhoxy en position 5, par condensation du chlorure de benzoyle, en présence de pyridine dans un hydrocarbure aliphatique chloré, de préférence le chloroforme ou le dichloréthane, à température ambiante.

15. À titre de composé intermédiaire, le composé de formule générale

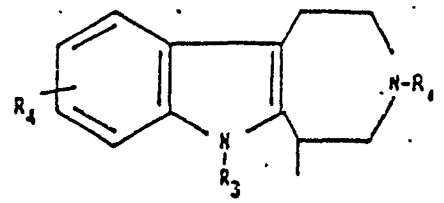

COO-Alkyl

dans laquelle les radicaux $R_1$,$R_3$ et $R_4$ ont les significations mentionnées dans la revendication 1 et les sels d'addition d'acides pharmaceutiquement acceptables.

16. À titre de composé intermédiaire, le composé de formule générale

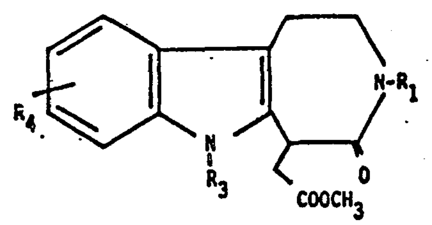

IX

dans laquelle $R_1$, $R_3$ et $R_4$ ont les significations mentionnées dans la revendication 1 et les sels d'addition d'acides pharmaceutiquement acceptables.

17. Préparation pharmaceutique contenant au moins l'un des dérivés selon la revendication 1 ou des sels d'addition d'acides pharmaceutiquement acceptables de ceux-ci, en combinaison avec des excipients classiques permettant, de préférence une administration par voie orale ou parentérale.

18. Préparation pharmaceutique selon la revendication 17 caractérisée en ce qu'elle contient le principe actif en une dose allant de 10 à 100 mg.

19. Utilisation des dérivés selon la revendication 1 et des sels d'addition d'acides de ceux-ci pour le traitement des troubles de la vigilance, en particulier pour lutter contre les troubles du comportement imputables à des dommages vasculaires cérébraux et à la sclérose cérébrale en gériatrie.